# EUROPEAN PATENT SPECIFICATION

(11) **EP 2 832 301 B1**
(45) Date of publication and mention of the grant of the patent: **05.10.2022**
(21) Application number: 13768700.0
(22) Date of filing: 25.03.2013
(51) Int. Cl.: A61B 8/12, A61B 8/08, A61B 5/00

(54) **PROBE AND DIAGNOSTIC IMAGING DEVICE**
SONDE UND DIAGNOSTISCHE BILDGEBUNGSVORRICHTUNG
SONDE ET DISPOSITIF D'IMAGERIE DIAGNOSTIQUE

(30) Priority: 28.03.2012 JP 2012072857
(43) Date of publication of application: 04.02.2015
(73) Proprietor: Terumo Kabushiki Kaisha, Tokyo 151-0072 (JP)
(72) Inventor: KANEKO, Kenji, Ashigarakami-gun Kanagawa 259-0151 (JP)
(74) Representative: Casalonga
(86) International application number: PCT/JP2013/002005
(87) International publication number: WO 2013/145689

(56) References cited:
- EP-A1- 2 401 951
- WO-A1-2008/086613
- WO-A1-2011/039983
- WO-A1-2014/077870
- WO-A2-2008/057573
- JP-A- 2010 207 612
- JP-A- 2010 508 973
- JP-A- 2010 516 304
- JP-A- 2011 519 687
- US-A1- 2005 101 859

## Description

### Technical Field

The present invention relates to a probe and an imaging apparatus for diagnosis.

### Background Art

Hitherto, imaging apparatuses for diagnosis have been widely used for performing diagnoses of arteriosclerosis, preoperative diagnoses during intra-vascular treatment using a high-performance catheter such as a balloon catheter or a stent, and checking postoperative results.

The imaging apparatus for diagnosis includes an intra-vascular ultra sound diagnostic apparatus (IVUS) and an optical coherence tomography diagnostic apparatus (OCT) and the like, each of which has characteristics different from each other.

Recently, an imaging apparatus for diagnosis has been proposed in which the function of the IVUS and the function of the OCT are combined (for example, see PTL 1). According to such an imaging apparatus for diagnosis, it is possible to generate a tomographic image taking advantage of the characteristics of the IVUS which can measure up to a high depth region and advantage of the characteristics of the OCT which can measure an area with a high resolution. In addition, there has been also proposed a technique of increasing obtainable tomographic images for each unit time (that is, image information is acquired at a high speed) by radiating the same signals (energy) in a plurality of directions (for example, see PTL 2).

Here, it is required to dispose a plurality of image sensors for one probe in an imaging apparatus for diagnosis in which the function of IVUS and the function of OCT are combined, and also an imaging apparatus for diagnosis which is intended to acquire image information at a high speed. However, it is difficult to dispose the plurality of image sensors on the spatially same positions, and observation positions of both sensors become different. Therefore, in PTL 1, there has been proposed a technique of correcting deviations of both sensors in the probe in a longer axial direction and deviations in a rotary direction.

### Citation List

### Patent Literature

PTL 1: JP-A-2005-152626
PTL 2: JP-A-2006-162485
WO 2008/057573 A2 and WO 2008/086613 A1 disclose imaging probes comprising imaging means both for optical coherence tomography (OCT) and intravascular ultrasound (IVUS).

### Summary of Invention

### Technical Problem

However, generally in the imaging apparatus for diagnosis described above, transmitting and receiving units transmit and receive ultrasound or light while causing a probe to perform a rotary operation and an axial-direction operation inside a blood vessel, and thereby the imaging apparatus generates tomographic images. Therefore, when the rotary operation and the axial-direction operation are performed, if a transmitting and receiving unit for IVUS and a transmitting and receiving unit for OCT are disposed to be inclined in different directions with respect to a direction orthogonal to the direction of the rotary axis (that is, if one is disposed to be inclined to a distal side of the probe and the other is disposed to be inclined to a proximal side of the probe), angles of cross sections cut on the ultrasonic tomographic image and the optical tomographic image are different.

In such cases, since the observation angles of the ultrasonic tomographic image and the optical tomographic image are different from each other, it is difficult to perform correction by image processing. Especially, in the case of a high resolution tomographic image, it is desirable to match the observation angles to some extent.

Further, with respect to the transmission angles of signals (energy) from respective image sensors, optimum angles are respectively determined according to the border conditions of the probe. When the respective signals are transmitted at different angles, if these signals intersect near the center of the observation scope (along the depth direction), it is possible to cut more closely observation cross sections. However, in such configurations, when the transmitting and receiving unit for IVUS and the transmitting and receiving unit for OCT are disposed to be inclined in directions different from each other with respect to the direction orthogonal to the direction of the rotary axis, the distance between the sensors becomes long. Therefore, the passing performance of the probe inside the blood vessel becomes bad.

The present invention has been made taking the aforementioned problems into consideration, and aims to provide a technique of matching observation angles in an imaging apparatus for diagnosis that can generate respective tomographic images by using a plurality of transmitting and receiving units.

### Solution to Problem

In order to solve the problem described above, an aspect of the invention is to provide a probe as defined in claim 1. Preferred embodiments are defined in the dependent claims.

### Advantageous Effects of Invention

According to the present invention, it is possible to match observation cross layers of respective tomographic images generated by using a plurality of transmitting and receiving units.

Other characteristics or advantages of the present invention are as described below with reference to the accompanied drawings. Note that, in the accompanied drawings, the same or similar configurations are denoted by the same reference numerals.

### Brief Description of Drawings

The drawings illustrate embodiments of the present invention.
[Fig. 1] Fig. 1 is a diagram illustrating an example of an entire configuration of an imaging apparatus for diagnosis 100 according to an embodiment of the present invention.
[Fig. 2] Fig. 2 is a diagram illustrating an example of the configuration of a probe 101 illustrated in Fig. 1.
[Fig. 3] Fig. 3 is a diagram illustrating an example of a cross-sectional configuration of an imaging core 220 illustrated in Fig. 2.
[Fig. 4A] Fig. 4A is a diagram illustrating an example of a positional relationship between an ultrasoundultrasound transmitting and receiving unit 310 and an optical transmitting and receiving unit 320.
[Fig. 4B] Fig. 4B is a diagram illustrating an example of a positional relationship between the ultrasound transmitting and receiving unit 310 and the optical transmitting and receiving unit 320.
[Fig. 5A] Fig. 5A is a diagram illustrating an example of energy transmission directions of the ultrasound transmitting and receiving unit 310 and the optical transmitting and receiving unit 320.
[Fig. 5B] Fig. 5B is a diagram illustrating an example of energy transmission directions of the ultrasound transmitting and receiving unit 310 and the optical transmitting and receiving unit 320.
[Fig. 6] Fig. 6 is a diagram illustrating an example of a positional relationship between the ultrasound transmitting and receiving unit 310 and the optical transmitting and receiving unit 320 in the rotary angle direction.
[Fig. 7A] Fig. 7A is a diagram illustrating an example of a positional relationship between the ultrasound transmitting and receiving unit 310 and the optical transmitting and receiving unit 320 in the rotary angle direction.
[Fig. 7B] Fig. 7B is a diagram illustrating an example of a positional relationship between the ultrasound transmitting and receiving unit 310 and the optical transmitting and receiving unit 320 in the rotary angle direction.
[Fig. 8] Fig. 8 is a diagram illustrating an example of a functional configuration of the imaging apparatus for diagnosis 100 illustrated in Fig. 1.
[Fig. 9] Fig. 9 is a diagram illustrating an example of a modification example.
[Fig. 10] Fig. 10 is a diagram illustrating an example of a modification example.
[Fig. 11A] Fig. 11A is a diagram illustrating an example of a modification example.
[Fig. 11B] Fig. 11B is a diagram illustrating an example of a modification example.

### Description of Embodiments

Hereinafter, exemplary embodiments of the present invention are described in detail with reference to the accompanying drawings.

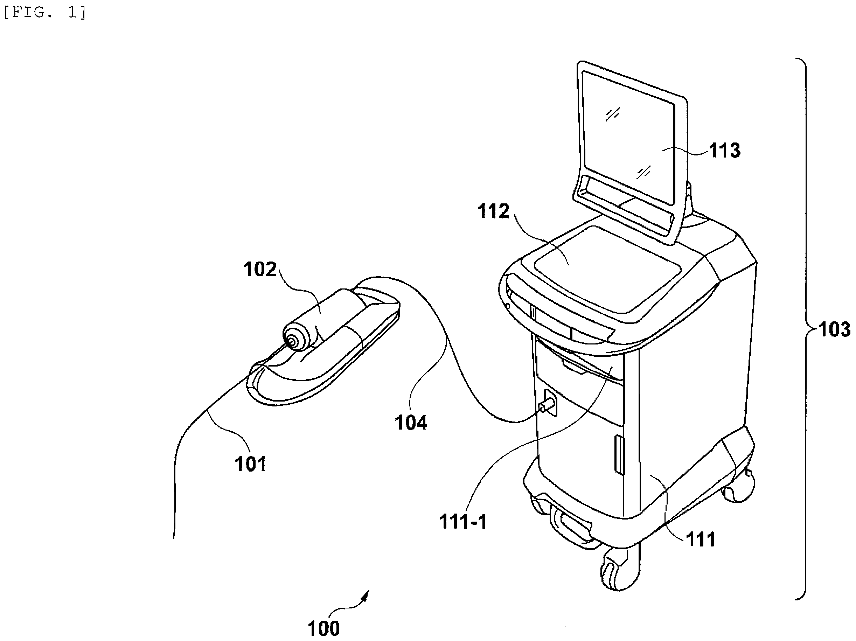

Fig. 1 is a diagram illustrating an example of an entire configuration of an imaging apparatus for diagnosis 100 according to an embodiment of the present invention.

The imaging apparatus for diagnosis 100 is configured to include a function of IVUS and a function of OCT. That is, the imaging apparatus for diagnosis 100 generates a tomographic image that utilizes both the characteristics of the IVUS that can measure a high depth region and the characteristics of the OCT that can measure an area with a high resolution.

Here, the imaging apparatus for diagnosis 100 is configured to include a probe 101, a scanner & pull-back unit 102, and an operation control apparatus 103, and the scanner & pull-back unit 102 and the operation control apparatus 103 are connected to each other through a signal line 104 so that various signals can be transmitted.

The probe 101 is directly inserted into the inside of a body lumen such as a blood vessel (hereinafter, simply referred to as "body cavity" in some cases), and an imaging core includes an ultrasound transmitting and receiving unit that transmits an ultrasound to the body cavity based on a pulse signal, and receives a reflected wave from the inside of the body cavity and an optical transmitting and receiving unit that continuously transmits transmitted light (measurement light) to the inside of the body cavity and continuously receives reflected light from the inside of the body cavity is inserted. The imaging apparatus for diagnosis 100 measures the state in the body cavity by using the imaging core as described above.

The scanner & pull-back unit (rotary junction unit) 102 is detachably attached to the probe 101, and defines an axial direction movement and a rotation direction movement of the imaging core inserted into the probe 101 in the body cavity by driving a built-in motor. Additionally, the scanner & pull-back unit 102 acquires the reflected wave received in the ultrasound transmitting and receiving unit and the reflected light received in the optical transmitting and receiving unit, and transmits the reflected wave and the reflected light to the operation control apparatus 103.

The operation control apparatus 103 has a function of collectively controlling operations of the imaging apparatus for diagnosis 100. For example, the operation control apparatus 103 has a function of inputting various set values based on a user' s instructions into the apparatus and a function of processing data obtained by the measurement and displaying the data as tomographic images of the inside of the body cavity.

The operation control apparatus 103 is provided with a main body control unit 111, a printer & DVD recorder 111-1, an operation panel 112, an LCD monitor 113, and the like. The main body control unit 111 generates an ultrasonic tomographic image or an optical tomographic image. The ultrasonic tomographic image is generated by the ultrasound data based on the reflected wave obtained by the measurement, and is generated by processing the line data generated based on the ultrasound data. Further, the optical tomographic image is generated by generating interference light data by interfering with the reflected light obtained by the measurement and reference light obtained by separating light from a light source and by processing line data generated based on the interference light data.

The printer & DVD recorder 111-1 prints process results in the main body control unit 111 or stores the process results as data. The operation panel 112 is a user interface through which a user inputs various set values and instructions. The LCD monitor 113 functions as a display apparatus, and displays, for example, the tomographic image generated in the main body control unit 111.

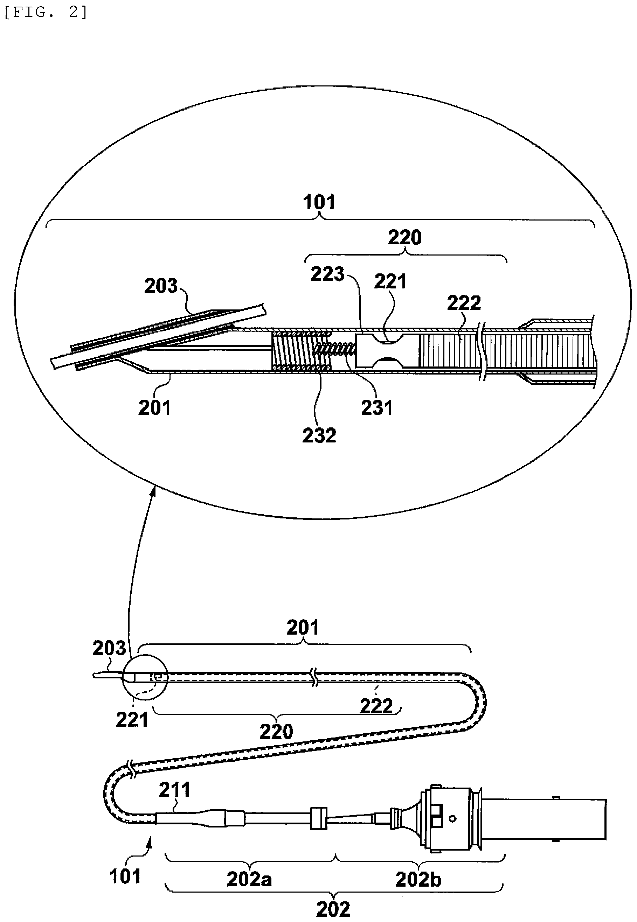

Next, an exemplary configuration of the probe 101 illustrated in Fig. 1 is described with reference to Fig. 2. Fig. 2 is a diagram illustrating the entire configuration of the probe 101 and a cross-sectional configuration of a distal end portion.

The probe 101 includes a long catheter sheath 201 to be inserted into the inside of the body cavity such as a blood vessel, and a connector unit 202 that is not inserted into the inside of the body cavity such as a blood vessel, and is disposed near the user's hand side so as to be operated by the user.

A tube for guide wire lumen 203 that configures a guide wire lumen is provided on the distal end of the catheter sheath 201. The catheter sheath 201 forms a lumen that continues from the connection section with the tube for guide wire lumen 203 to the connection section with the connector unit 202.

An imaging core 220 that includes a transmitting and receiving unit 221 and a coil-shaped drive shaft 222 is inserted into the inside portion of the lumen of the catheter sheath 201 throughout almost the entire length of the catheter sheath 201. The transmitting and receiving unit 221 is provided with the ultrasound transmitting and receiving unit for transmitting and receiving an ultrasound and the optical transmitting and receiving unit for transmitting and receiving light. In addition, the drive shaft 222 includes an electric signal cable and an optical fiber cable in the inside portion and transfers a rotary-drive force for rotating the same.

The connector unit 202 includes a sheath connector 202a that is configured on a proximal end of the catheter sheath 201 in an integrated manner and a drive shaft connector 202b that is configured on a proximal end of the drive shaft 222 in a manner of rotatably fixing the drive shaft 222.

An anti-kink protector 211 is provided on the boundary portion between the sheath connector 202a and the catheter sheath 201. According to this, the boundary portion maintains a predetermined rigidity, and bending (kinks) caused by the rapid change of physical properties can be prevented. In addition, the proximal end of the drive shaft connector 202b is detachably attached to the scanner & pull-back unit 102.

Next, the cross-sectional configuration of the distal end portion of the probe 101 is described. The imaging core 220 including a housing 223 that has the transmitting and receiving unit 221 in which the ultrasound transmitting and receiving unit that transmits and receives the ultrasound and the optical transmitting and receiving unit that transmits and receives light are disposed, and the drive shaft 222 that transfers a rotary-drive force for rotating the transmitting and receiving unit 221 is inserted into the inside portion of the lumen of the catheter sheath 201, throughout the entire length, so as to form the probe 101.

The transmitting and receiving unit 221 transmits an ultrasound and measurement light to a tissue in the body cavity, and receives a reflected wave and reflected light from the tissue in the body cavity.

The drive shaft 222 is formed in a coil shape, and an electric signal cable and an optical fiber cable (an optical fiber cable in a single mode) are provided in the inside portion of the drive shaft 222.

The housing 223 has a shape having notches in a portion of a short cylindrical metal pipe, and is formed by performing carving or metal powder injection molding (MIM) on a metal ingot. The housing 223 has the ultrasound transmitting and receiving unit and the optical transmitting and receiving unit in the inside portion as the transmitting and receiving unit 221, and is connected to the drive shaft 222 on the proximal side. Additionally, a short coil-shaped elastic member 231 is provided on the distal side.

Since the elastic member 231 is obtained by forming a stainless steel wire into a coil shape and the elastic member 231 is provided on the distal side, when the imaging core 220 is moved back and forth, the imaging core 220 is prevented from being caught in the catheter sheath 201.

A reinforcement coil 232 is provided for the purpose of preventing the distal end portion of the catheter sheath 201 from being drastically bent.

The tube for guide wire lumen 203 has a lumen for a guide wire into which the guide wire can be inserted. The tube for guide wire lumen 203 is used for receiving the guide wire inserted into the body cavity such as the blood vessel in advance, and for causing the guide wire to guide the catheter sheath 201 to a target lesion.

The drive shaft 222 is able to cause the transmitting and receiving unit 221 to rotate and move in the axial direction with respect to the catheter sheath 201, and is configured with a multiplex and multilayer contact coil which has a characteristic of being flexible and capable of effectively transferring rotation, and is made with, for example, a metal wire such as stainless steel.

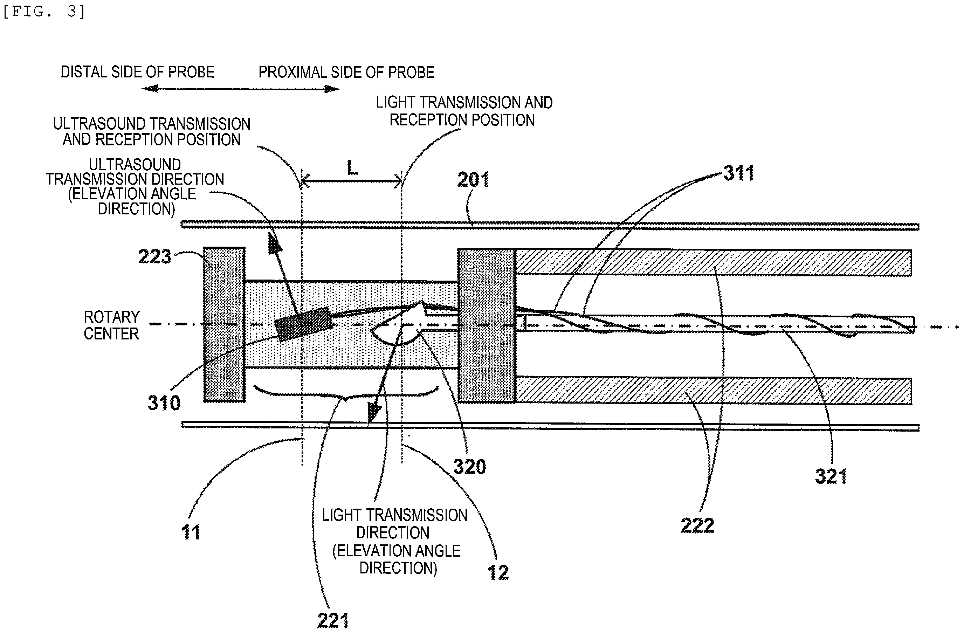

Next, an example of the cross-sectional configuration of the imaging core 220 illustrated in Fig. 2 is described with reference to Fig. 3.

The transmitting and receiving unit 221 provided in the housing 223 includes an ultrasound transmitting and receiving unit 310 and the optical transmitting and receiving unit 320, and the ultrasound transmitting and receiving unit 310 and the optical transmitting and receiving unit 320 each are disposed along the axial direction on the rotary central axis of the drive shaft 222 (on the alternate long and short dash line in Fig. 3).

Electric signal cables 311 connected to the ultrasound transmitting and receiving unit 310 and an optical fiber cable 321 connected to the optical transmitting and receiving unit 320 are disposed in the inside portion of the drive shaft 222, and the electric signal cables 311 are wound around the optical fiber cable 321 in a spiral shape.

The ultrasound transmitting and receiving unit 310 is disposed on the distal side of the probe 101 and the optical transmitting and receiving unit 320 is disposed on the proximal side of the probe 101, and the ultrasound transmitting and receiving unit 310 and the optical transmitting and receiving unit 320 are attached in the housing 223 so that a distance between an ultrasound transmission and reception position of the ultrasound transmitting and receiving unit 310 and a light transmission and reception position of the optical transmitting and receiving unit 320 is L.

Additionally, the ultrasound transmitting and receiving unit 310 and the optical transmitting and receiving unit 320 are attached in the housing 223 so that the ultrasound transmission direction (elevation angle direction) of the ultrasound transmitting and receiving unit 310 and the light transmission direction (elevation angle direction) of the optical transmitting and receiving unit 320 respectively are formed at about 90° with respect to the axial direction of the drive shaft 222.

Here, when the ultrasound transmitting and receiving unit 310 and the optical transmitting and receiving unit 320 are disposed to be formed at 90° with respect to the axial direction of the drive shaft 222, the intensity of the reflected light from the catheter sheath 201 is strong, and the influence of the reflected noise on the obtained tomographic image is strong. Therefore, it is desirable that the transmitting and receiving units be disposed to have inclinations toward any one of the distal side or the proximal side of the probe 101.

Further, if the transmitting and receiving units are respectively disposed to be inclined in different directions (to the distal side of the probe 101 and to the proximal side of the probe 101), the angles of the observation cross sections cut in the ultrasonic tomographic image and the optical tomographic image become different, and the inconsistency of the observation cross sections is generated.

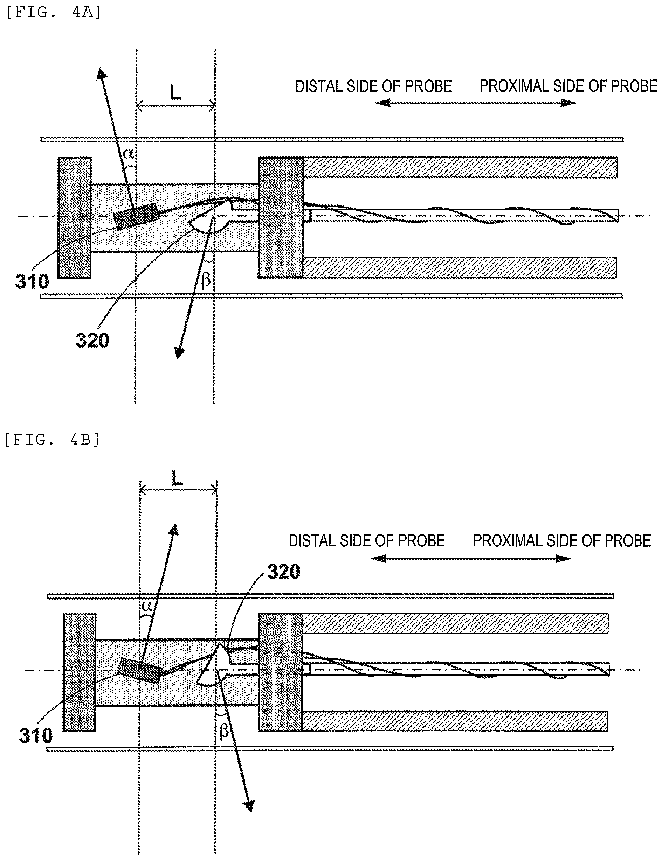

Therefore, according to the present embodiment, the transmitting and receiving units are disposed to have inclinations in the same direction along the axial direction of the drive shaft 222. More specifically, as illustrated in Fig. 4A, the ultrasound transmitting and receiving unit 310 is disposed to be inclined by α° with respect to a direction orthogonal to the axial direction of the drive shaft 222 toward the distal side of the probe 101, and the optical transmitting and receiving unit 320 is disposed to be inclined by β° with respect to a direction orthogonal to the axial direction of the drive shaft 222 toward the distal side of the probe 101.

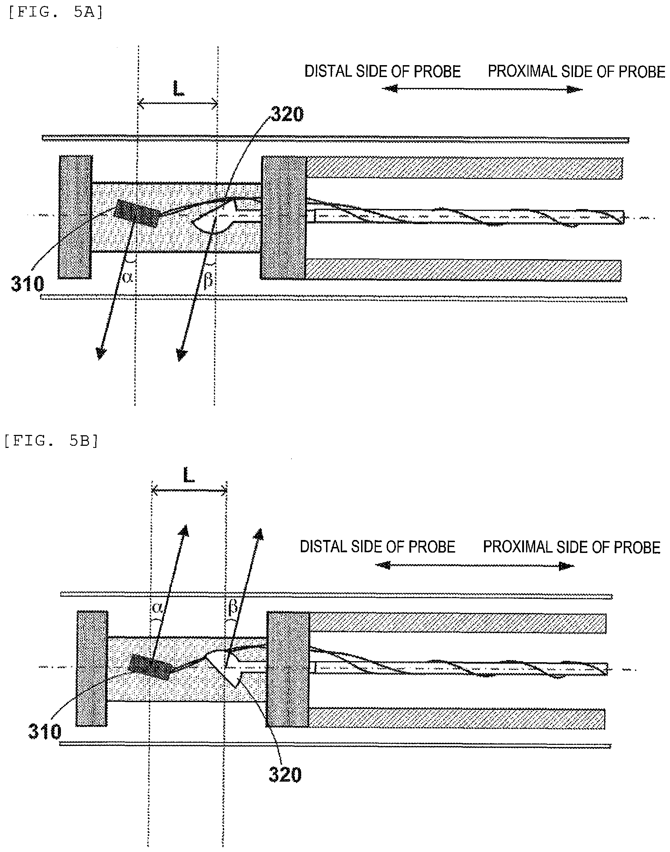

Note that, according to the present embodiment, α and β are the same value. Here, for easier understanding of the description, it is illustrated that angles of α and β are great (in the same manner with respect to Figs. 4B, 5A, and 5B to be described below), but α and β are set to be 4°, 8°, or the like, in reality.

Of course, the disposition configuration of the transmitting and receiving units is not limited to the configuration illustrated in Fig. 4A, and for example, as illustrated in Fig. 4B, the ultrasound transmitting and receiving unit 310 may be disposed to be inclined by α° in the direction orthogonal to the axial direction of the drive shaft 222 toward the proximal side of the probe 101, or the optical transmitting and receiving unit 320 may be disposed to be inclined by β° in the direction orthogonal to the axial direction of the drive shaft 222 toward the proximal side of the probe 101.

Furthermore, the ultrasound transmitting and receiving unit 310 and the optical transmitting and receiving unit 320 may be disposed in the configuration illustrated in Fig. 5A or 5B. Even in the cases of Figs. 4B, 5A, and 5B, since the ultrasound transmitting and receiving unit 310 and the optical transmitting and receiving unit 320 are disposed to be inclined in the same direction along the axial direction of the drive shaft 222, the angles of the observation cross sections cut in the ultrasonic tomographic image and the optical tomographic image are caused to be as similar as possible in the same manner as the case of Fig. 4A. According to this, it is possible to cause the observation cross sections of both images to coincide with each other.

Next, an example of the positional relationship between the ultrasound transmitting and receiving unit 310 and the optical transmitting and receiving unit 320 with respect to a surface substantially orthogonal to the rotary axis of the drive shaft 222 is described.

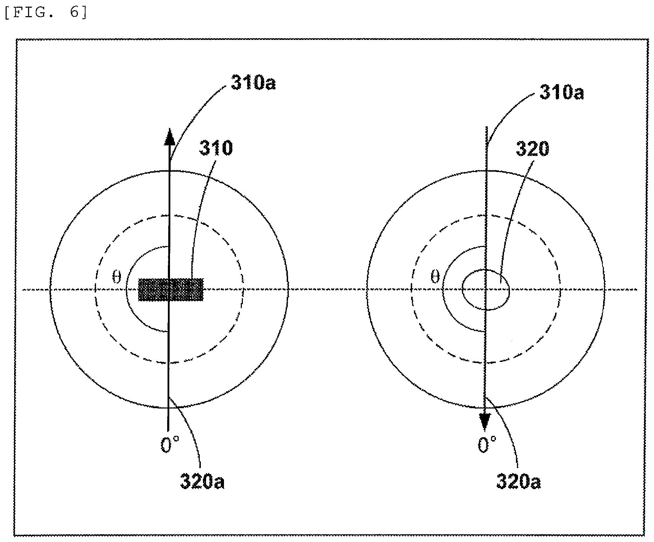

Here, Fig. 6 is a diagram illustrating the positional relationship between the ultrasound transmitting and receiving unit 310 and the optical transmitting and receiving unit 320 with respect to the surface substantially orthogonal to the rotary axis of the drive shaft 222. Note that, in all drawings, diagrams on the left side illustrate cross-sectional configurations when an ultrasound transmission and reception position 11 is cut with respect to the surface substantially orthogonal to the rotary central axis, and diagrams on the right side illustrate cross-sectional configurations when a light transmission and reception position 12 is cut with respect to the surface substantially orthogonal to the rotary central axis.

In this case, the ultrasound transmitting and receiving unit 310 and the optical transmitting and receiving unit 320 are disposed on the rotary central axis. A light transmission direction (rotary angle direction) 320a indicates a progress direction of light transmitted from the optical transmitting and receiving unit 320 when the light transmission and reception position 12 is cut with respect to the surface substantially orthogonal to the rotary central axis, and an ultrasound transmission direction (rotary angle direction) 310a indicates a progress direction of the ultrasound transmitted from the ultrasound transmitting and receiving unit 310 when an ultrasound transmission and reception position 11 is cut with respect to the surface substantially orthogonal to the rotary central axis.

Here, when the light transmission direction 320a is set to be 0, the ultrasound transmission direction (rotary angle direction) of the ultrasound transmitting and receiving unit 310 becomes θ. That is, the ultrasound transmitting and receiving unit 310 and the optical transmitting and receiving unit 320 are disposed to be deviated by θ (in this case, 180°) from each other along the rotary angle direction.

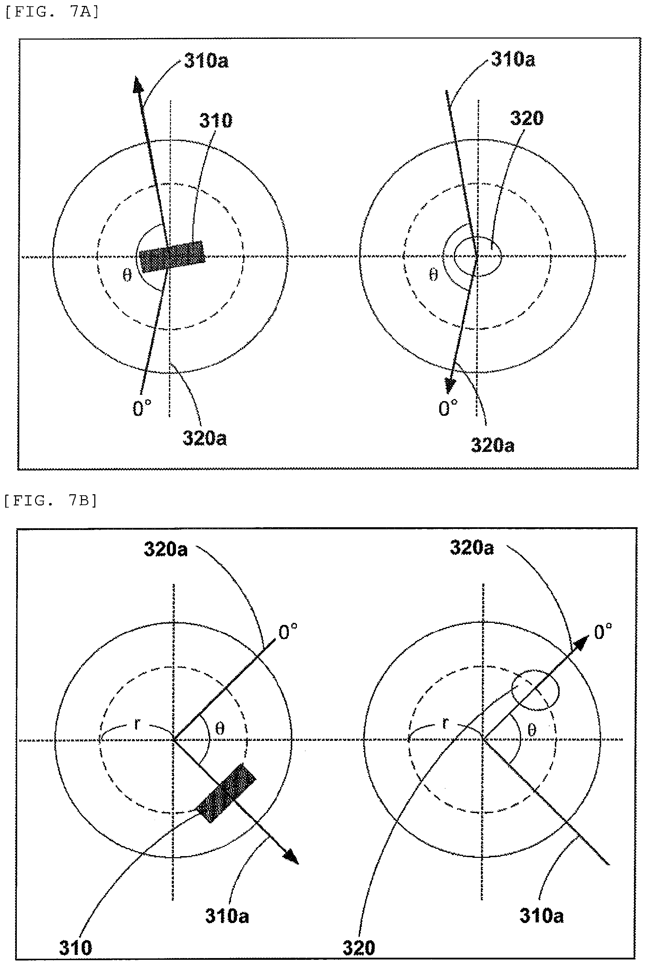

Note that, according to the present embodiment, the ultrasound transmission direction 310a of the ultrasound transmitting and receiving unit 310 and the light transmission direction 320a of the optical transmitting and receiving unit 320 may have the positional relationship with respect to the rotary angle direction, and the ultrasound transmitting and receiving unit 310 and the optical transmitting and receiving unit 320 may be disposed, for example, in the positional relationship as illustrated in Fig. 7A.

In the configurations illustrated in Figs. 6 and 7A as described above, the optical transmitting and receiving unit 320 and the ultrasound transmitting and receiving unit 310 are disposed on the same axis (on the rotary central axis) . This is to cause the image center of the constructed ultrasonic tomographic image and the image center of the optical tomographic image to coincide with each other. Note that, it is not necessary that the transmitting and receiving units be disposed on the same axis, and for example, as illustrated in Fig. 7B, the transmitting and receiving unit may be disposed at a position separated from the rotary central axis by a distance r. In the case of the positional relationship, since the respective image centers may be adjusted by the image processing when the ultrasound image is generated and when the optical tomographic image is generated, the image center of the ultrasonic tomographic image and the image center of the optical tomographic image coincide with each other in the same manner when the transmitting and receiving units are disposed on the same axis.

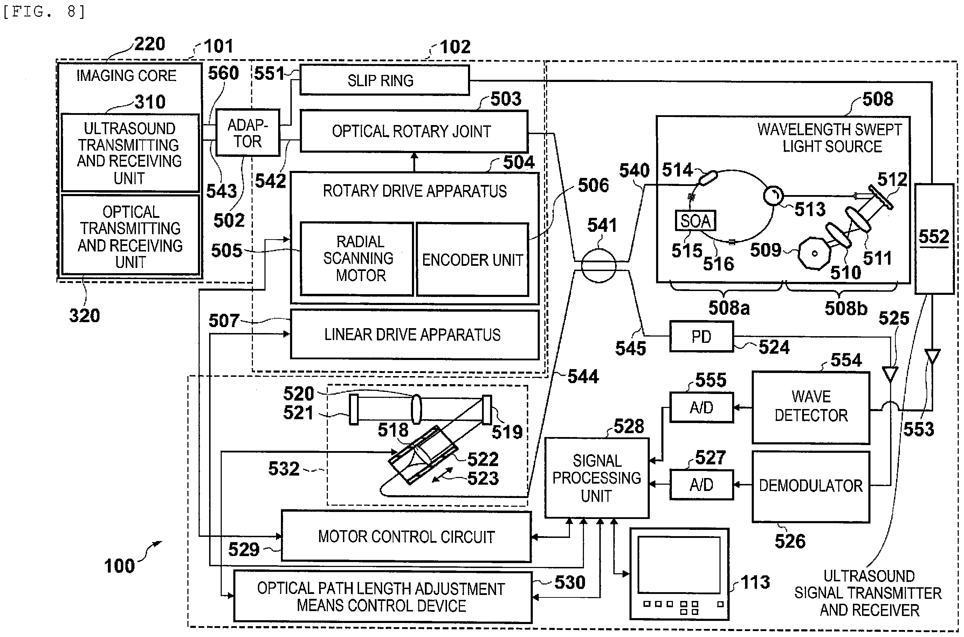

Next, an example of a functional configuration of the imaging apparatus for diagnosis 100 illustrated in Fig. 1 is described with reference to Fig. 8. As described above, the imaging apparatus for diagnosis 100 is realized in the configuration of combining the function of IVUS and the function of OCT (here, wavelength sweeping-type OCT as an example). Note that, since the imaging apparatus for diagnosis combining the function of IVUS and another function of OCT also has the same functional configuration, the description thereof is omitted here.

(1) First, a functional configuration corresponding to the function of IVUS is described.

The imaging core 220 includes the ultrasound transmitting and receiving unit 310 in the inside of the distal end, and the ultrasound transmitting and receiving unit 310 transmits an ultrasound to a biological tissue based on a pulse wave transmitted from an ultrasound signal transmitter and receiver 552, receives a reflected wave (echo) thereof, and transmits the reflected wave to the ultrasound signal transmitter and receiver 552 as an ultrasound echo through an adaptor 502 and a slip ring 551.

Note that, a rotary-drive unit side of the slip ring 551 is rotationally driven by a radial scanning motor 505 of a rotary-drive apparatus 504. Additionally, a rotary angle of the radial scanning motor 505 is detected by an encoder unit 506. Further, the scanner & pull-back unit 102 includes a linear drive apparatus 507, and defines a movement of the imaging core 220 in an axial direction based on a signal from a signal processing unit 528.

The ultrasound signal transmitter and receiver 552 includes a transmission wave circuit and a reception wave circuit (not illustrated). The transmission wave circuit transmits a pulse wave to the ultrasound transmitting and receiving unit 310 in the imaging core 220 based on a control signal transmitted from the signal processing unit 528. In addition, the reception wave circuit receives an ultrasound signal from the ultrasound transmitting and receiving unit 310 in the imaging core 220. After the received ultrasound signal is amplified by an amplifier 553, the received ultrasound signal is input to a wave detector 554 to be detected.

Further, in an A/D converter 555, an ultrasound signal output from the wave detector 554 is sampled at as many as 200 points at 30.6 MHz to generate digital data (ultrasound data) of 1 line. Note that, here, the frequency is set to be 30.6 MHz, but the frequency is calculated on an assumption that 200 points are to be sampled with respect to the depth of 5 mm when the sound velocity is considered to be 1,530 m/sec. Therefore, the sampling frequency is not particularly limited thereto.

The ultrasound data in a line unit generated in the A/D converter 555 is input to the signal processing unit 528. In the signal processing unit 528, the ultrasound data is converted into a gray scale so as to form the ultrasound tomographic image in each position inside a body cavity such as a blood vessel, and is output to the LCD monitor 113 at a predetermined frame rate.

Note that, the signal processing unit 528 is connected to a motor control circuit 529, and receives a video synchronization signal of the motor control circuit 529. In the signal processing unit 528, the ultrasound tomographic image is constructed by being synchronized with the received video synchronization signal. Additionally, the video synchronization signal of the motor control circuit 529 is also transmitted to the rotary-drive apparatus 504, and the rotary-drive apparatus 504 outputs a drive signal which is synchronized with the video synchronization signal.

(2) Subsequently, a functional configuration with respect to the function of the wavelength sweeping-type OCT is described.

A wavelength swept light source (swept laser) 508 is a type of an extended-cavity laser which is configured to have an optical fiber 516 which is coupled with a semiconductor optical amplifier 515 (SOA) in a ring shape, and a polygon scanning filter (508b).

Light output from the SOA 515 proceeds through the optical fiber 516 and is input to the polygon scanning filter 508b, and the light is subjected to wavelength selection herein, is amplified in the SOA 515, and lastly, is output from a coupler 514.

In the polygon scanning filter 508b, the wavelength is selected by combining a diffraction grating 512 which performs light-splitting, and a polygon mirror 509. Specifically, rays which is light-split by the diffraction grating 512 are concentrated on a surface of the polygon mirror 509 by using two lenses (510, 511). Accordingly, only the light in a wavelength which is orthogonal to the polygon mirror 509 returns to the same optical path, and is output from the polygon scanning filter 508b. That is, time sweeping of a wavelength can be performed by rotating the polygon mirror 509.

In the polygon mirror 509, for example, a 32-hedron mirror is used and the number of rotations is approximately 50,000 rpm. High speed and high output wavelength sweeping can be performed through the wavelength sweeping method in which the polygon mirror 509 and the diffraction grating 512 are combined.

Light of the wavelength swept light source 508 output from the coupler 514 is incident on an end of a first single mode fiber 540, and is transmitted to the distal side thereof. The first single mode fiber 540 is optically coupled to a second single mode fiber 545, a third single mode fiber 544, and a sixth single mode fiber 546 in a photo coupler unit 541 which are in the intermediate portion.

In the sixth single mode fiber 546 on a further distal side than the photo coupler unit 541 of the first single mode fiber 540, an optical rotary joint (optical coupling portion) 503 which connects a non-rotary portion (fixing portion) and a rotary portion (rotary-drive portion) with each other and transfers light is provided inside the rotary-drive apparatus 504.

Further, on a distal side of a fourth single mode fiber 542 inside the optical rotary joint (optical coupling portion) 503, a fifth single mode fiber 543 of the probe 101 is freely and detachably connected via the adaptor 502. Accordingly, light from the wavelength swept light source 508 is transferred to the fifth single mode fiber 543 which is inserted through the imaging core 220 to be rotatably driven.

Transferred light is radiated while performing the radial scanning from the light transmitting and receiving unit 320 of the imaging core 220 to a biological tissue inside a body cavity. Then, a portion of reflected light scattered in the inside or on a surface of a biological tissue is collected by the light transmitting and receiving unit 320 of the imaging core 220, and returns to the first single mode fiber 540 side via an optical path in reverse. Further, the portion of the reflected light moves to the second single mode fiber 545 side by the photo coupler unit 541, and the light is received by the light detector (for example, photo-diode 524) after being emitted from an end of the second single mode fiber 545.

Note that, the rotary-drive portion side of the optical rotary joint 503 is rotationally driven by the radial scanning motor 505 of the rotary-drive apparatus 504. Additionally, a rotary angle of the radial scanning motor 505 is detected by the encoder unit 506. Further, the scanner & pull-back unit 102 includes the linear drive apparatus 507 and defines the axial-direction operation of the imaging core 220 based on an instruction from the signal processing unit 528.

Meanwhile, an optical path length varying mechanism 532 for fine-adjusting the optical path length of the reference light is provided in a distal end on a side opposite to the photo coupler unit 541 of the third single mode fiber 544.

The optical path length varying mechanism 532 includes optical path length changing means for changing the optical path length corresponding to a fluctuation in a length of each probe 101 so as to be able to absorb the fluctuation in the length thereof when the probe 101 is replaced and used.

The third single mode fiber 544 and a collimating lens 518 are provided on a one-axis stage 522 which is movable in an optical-axis direction as indicated by the arrow 523, and forms the optical path length changing means.

Specifically, the one-axis stage 522 functions as the optical path length changing means having a movable range of the optical path length as wide as the fluctuation in the optical path length of the probe 101 can be absorbed when the probe 101 is replaced. Further, the one-axis stage 522 also includes a function as adjustment means for adjusting an offset. For example, when the distal end of the probe 101 is not in close contact with a surface of a biological tissue, it is possible to set a state of being interfered with the reflected light from the surface position of the biological tissue by minutely changing the optical path length through the one-axis stage.

The optical path length is fine-adjusted in the one-axis stage 522, and light reflected by the mirror 521 via a grating 519 and a lens 520 is mixed with light acquired from the sixth single mode fiber 546 side in the photo coupler unit 541 which is provided in an intermediate portion of the third single mode fiber 544, and thus, the light is received in the photo-diode 524.

The interference light received in the photo-diode 524 in this manner is subjected to photoelectric conversion, thereby being input to a demodulator 526 after being amplified by the amplifier 525. In the demodulator 526, demodulation processing in which only the signal portion is extracted from the interfered light is performed, and the output is input to an A/D converter 527 as an interference light signal.

In the A/D converter 527, an interference light signal is sampled, for example, at as many as 2,048 points at 180 MHz, thereby generating digital data (interference light data) of 1 line. Note that, the sampling frequency is set to 180 MHz on a premise that approximately 90% of a periodical cycle (12.5 µsec) of the wavelength sweep is extracted as digital data of 2,048 points when a repetition frequency of the wavelength sweep is set to 40 kHz, without being particularly limited thereto.

The interference light data in a line unit generated in the A/D converter 527 is input to the signal processing unit 528. When in a measurement mode, the interference light data is subjected to frequency resolution through Fast Fourier Transform (FFT) in the signal processing unit 528 so as to generate data in a depth direction (line data), and the line data is subjected to coordinate-conversion to construct a light tomographic image in each position inside a body cavity such as a blood vessel, thereby being output to the LCD monitor 113 at a predetermined frame rate.

The signal processing unit 528 is further connected to an optical path length adjustment means control device 530. The signal processing unit 528 controls a position of the one-axis stage 522 via the optical path length adjustment means control device 530.

According to the process, the ultrasonic tomographic image and the optical tomographic image are displayed on the imaging apparatus for diagnosis 100 (the LCD monitor 113) toward the user. Note that, as a display mode of the tomographic images, respective tomographic images corresponding to respective positions in the axial direction of the body cavity such as a blood vessel may be displayed in parallel, or may be displayed to be overlapped so that the image centers coincide, without being particularly limited thereto.

According to the present embodiment described above, the ultrasound transmitting and receiving unit 310 and the optical transmitting and receiving unit 320 are disposed to be inclined in the same direction along the axial direction of the drive shaft 222. According to this, the angles of the observation cross sections (cut cross section) according to the ultrasonic tomographic image and the optical tomographic image can be formed as similar as possible.

Though the representative examples are described above, the present invention is not limited to the embodiments described above and the accompanying drawings and can be appropriately modified and practiced in the scope of the invention as defined in the appended claims.

Here, some modification examples are described.

### (Modification example 1)

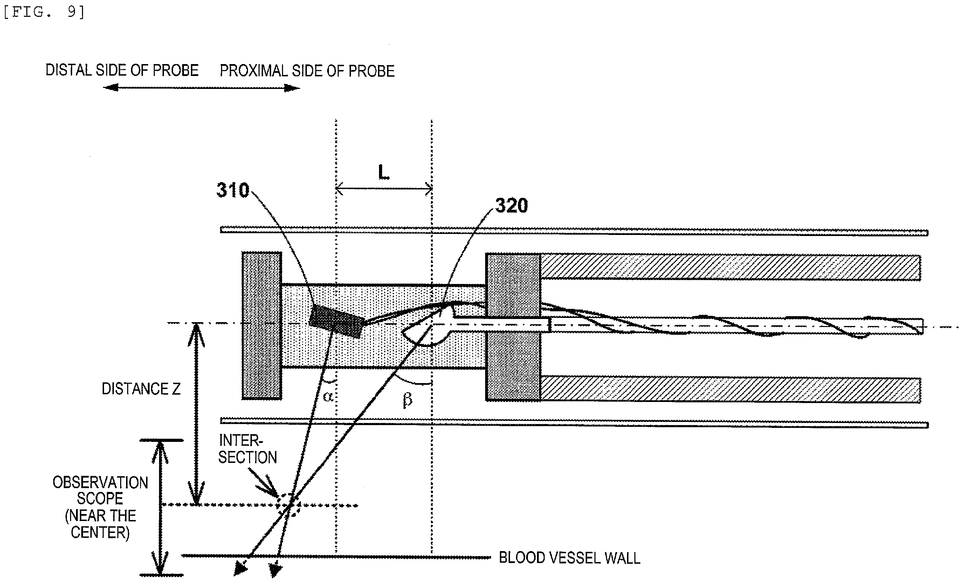

For example, in the embodiments described above, a case in which the ultrasound transmitting and receiving unit 310 and the optical transmitting and receiving unit 320 are disposed to have the same angles with respect to the direction orthogonal to the axial direction of the drive shaft 222 is described (α=β), but the present invention is not limited thereto. For example, as illustrated in Fig. 9, the inclination angle of the optical transmitting and receiving unit 320 may be greater than the inclination angle of the ultrasound transmitting and receiving unit 310 (α<β) . In this case, since the light transmitted from the optical transmitting and receiving unit 320 provided on the proximal side of the probe 101 intersects with the ultrasound transmitted from the ultrasound transmitting and receiving unit 310 provided on the distal side of the probe 101 near the intermediate portion of the observation scopes (positions separated from the rotary axis by a distance Z), it is possible to obtain the tomographic images at substantially the same positions.

Note that, there are optimum inclinations (α,β) for respective signals (light and ultrasound). Therefore, a distance between sensors of respective signals (distance between the ultrasound transmitting and receiving unit 310 and the optical transmitting and receiving unit 320) is unambiguously determined by the optimum angles. Since a configuration in which the sensors intersect in the same direction along the axial direction is employed, the distance between the sensors becomes shorter than in the configuration in which the sensors intersect in the different directions along the axial direction.

### (Modification example 2)

In addition, according to the embodiments described above, a case in which the ultrasound transmitting and receiving unit 310 is disposed on the distal side and the optical transmitting and receiving unit 320 is disposed on the proximal side is described, but the invention is not limited thereto. That is, the optical transmitting and receiving unit 320 may be disposed on the distal side, and the ultrasound transmitting and receiving unit 310 may be disposed on the proximal side.

### (Modification example 3)

The configuration described in Modification example 1 may be applied to the configuration described in Modification example 2. Specifically, when the ultrasound transmitting and receiving unit 310 and the optical transmitting and receiving unit 320 are disposed to be inclined to the distal side of the probe 101, the inclination angle of the ultrasound transmitting and receiving unit 310 provided on the proximal side of the probe 101 may be greater than the inclination angle (with respect to the orthogonal direction to the axial direction) of the optical transmitting and receiving unit 320 provided on the distal side of the probe 101.

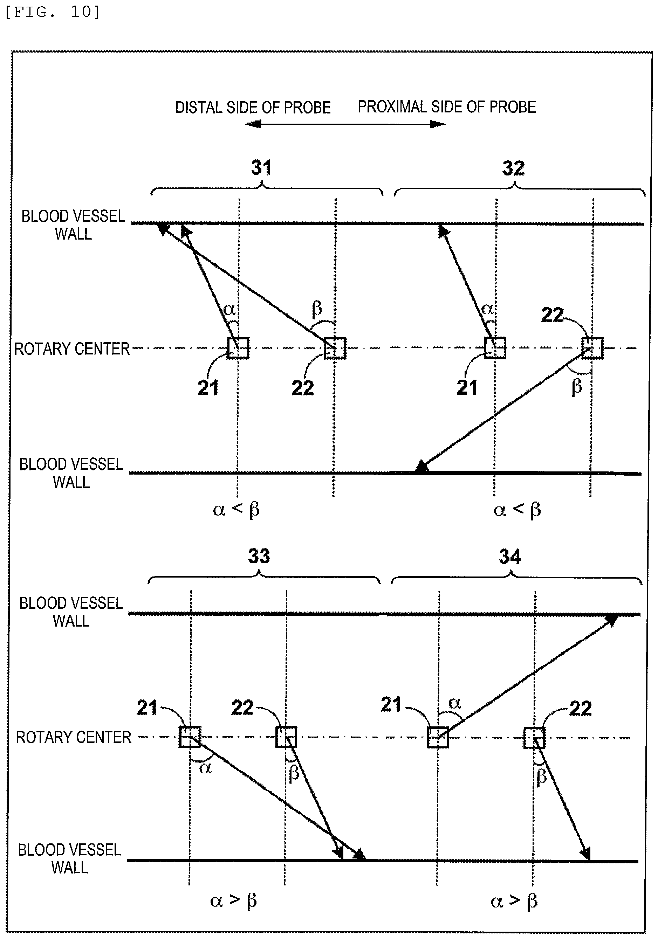

The variations are simply described with reference to Fig. 10. Here, description is made of setting the transmitting and receiving unit provided on the distal side of the probe 101 to be a first transmitting and receiving unit 21, and setting the transmitting and receiving unit provided on the proximal side of the probe 101 to be a second transmitting and receiving unit 22.

When the first transmitting and receiving unit 21 and the second transmitting and receiving unit 22 are provided to be inclined to the distal side of the probe 101, the angles of the first transmitting and receiving unit 21 and the second transmitting and receiving unit 22 may satisfy α<β, as indicated by Reference number 31. In addition, as indicated by Reference number 32, even if the rotary angle directions of the first transmitting and receiving unit 21 and the second transmitting and receiving unit 22 are different from each other by approximately 180°, the angles of the first transmitting and receiving unit 21 and the second transmitting and receiving unit 22 may be α<β in the same manner as in the description of Reference number 31.

Otherwise, if the first transmitting and receiving unit 21 and the second transmitting and receiving unit 22 are provided to be inclined to the proximal side of the probe 101, the angles of the first transmitting and receiving unit 21 and the second transmitting and receiving unit 22 may be α>β as indicated by Reference number 33. Further, as indicated by Reference number 34, even if the rotary angle directions of the first transmitting and receiving unit 21 and the second transmitting and receiving unit 22 are different from each other by approximately 180°, the angles of the first transmitting and receiving unit 21 and the second transmitting and receiving unit 22 may be α>β in the same manner as in the description of Reference number 33.

### (Modification example 4)

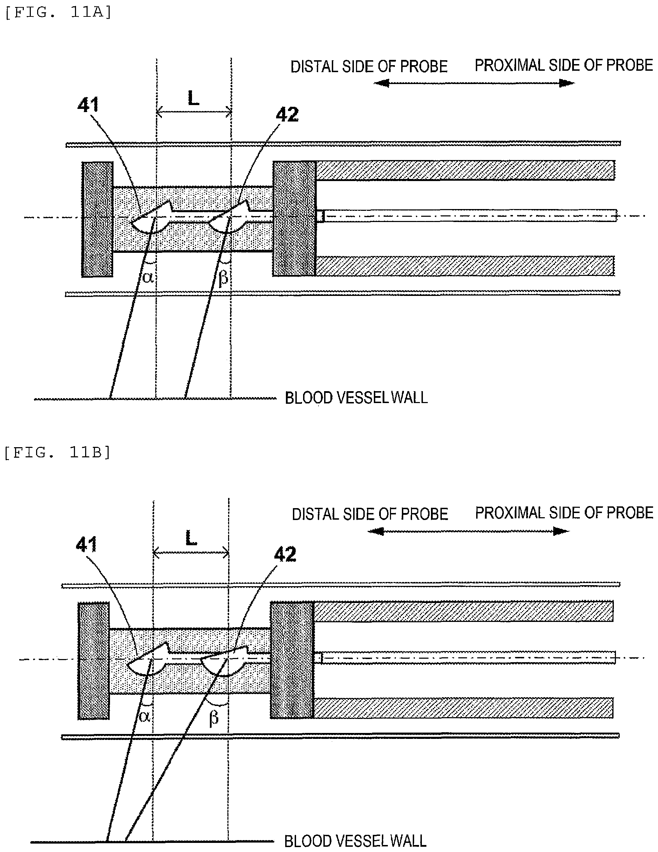

Further, in the embodiment described above, as an example of a configuration in which the plurality of transmitting and receiving units are provided in the probe, a case in which the ultrasound transmitting and receiving unit and the optical transmitting and receiving unit are provided in the probe is described, but the present invention is not limited thereto. For example, as illustrated in Fig. 11(a), the plurality of optical transmitting and receiving units may be provided. Note that, in this case, a plurality of optical fiber cables corresponding to respective optical transmitting and receiving units are also provided.

In the configuration of Fig. 11(a), since the same signals, that is, the same energies (in this case, light) are transmitted to the plurality of directions, the tomographic images obtained for each unit time increase, and the tomographic images are obtained at a high speed. Note that, an optical transmitting and receiving unit 41 and an optical transmitting and receiving unit 42 may be disposed to be inclined in the same directions along the axial direction, and the angles (α,β) with respect to the direction orthogonal to the axial direction may be the same or may be different from each other (in this case, the inclination of the optical transmitting and receiving unit 42 (that is, β) is set to be great) as illustrated in Fig. 11(b). In the configuration in which the same signals are transmitted in the plurality of directions, since it is desirable that different observation cross sections are acquired by respective transmitting and receiving units, the angles of the transmitting and receiving units may be adjusted in this regard.

Note that, herein, a case in which the plurality of optical transmitting and receiving units are provided is described, but it is obvious that the plurality of ultrasound transmitting and receiving units may be provided. In this case, in the same manner as described above, the tomographic images can be obtained at a high speed.

The present invention is not limited to the embodiments described above, and various modifications and changes are possible without departing from the scope of the present invention as defined in the following claims.

## Claims

1. A probe (101) configured to be detachably attached to a rotary junction unit (102) of an imaging apparatus for diagnosis (100) and insertable into an inside of a body lumen, comprising:
a plurality of transmitting and receiving units that are configured to obtain tomographic images by transmitting and receiving signals inside the body lumen; and
a drive shaft (222) configured to receive a rotary-drive force from the rotary junction unit (102) and to rotate the plurality of transmitting and receiving units,
wherein the plurality of transmitting and receiving units are respectively disposed along the axial direction of the drive shaft (222) inclined in the same direction by angles (α, β) with respect to a direction orthogonal to the axial direction of the drive shaft (222) so that the signals are transmitted in directions either to a distal side of the probe (101) or a proximal side of the probe (101),
wherein each of the plurality of transmitting and receiving units is selected from an ultrasound transmitting and receiving unit (310) that transmits and receives an ultrasound and an optical transmitting and receiving unit (320) that transmits and receives light,
wherein one of the plurality of transmitting and receiving units (310, 320) is disposed on the distal side of the probe (101) and the other of the plurality of transmitting and receiving units (310, 320) is disposed on the proximal side of the probe (101) so that a distance between them (310, 320) is L.

2. The probe (101) according to Claim 1,
wherein when the plurality of transmitting and receiving units are disposed by the angles so that the signals are transmitted to the distal side of the probe (101), the angle of the transmitting and receiving unit disposed on the proximal side of the probe (101) is equal to or greater than the angle of the other transmitting and receiving unit provided on the distal side of the probe (101), and
wherein when the plurality of transmitting and receiving units are disposed by the angles so that the signals are transmitted to the proximal side of the probe (101), the angle of the transmitting and receiving unit disposed on the distal side of the probe (101) is equal to or greater than the angle of the other transmitting and receiving unit provided on the proximal side of the probe (101).

3. The probe (101) according to Claim 1 or 2,
wherein the angles of the plurality of transmitting and receiving units are identical.

4. The probe (101) according to Claim 2,
wherein when the plurality of transmitting and receiving units are disposed by the angles to the distal side of the probe (101), the angle of the transmitting and receiving unit disposed on the proximal side of the probe (101) is greater than the angle of the other transmitting and receiving unit disposed on the distal side of the probe (101), and
wherein when the plurality of transmitting and receiving units are disposed by the angles to the proximal side of the probe (101), the angle of the transmitting and receiving unit disposed on the distal side of the probe (101) is greater than the angle of the other transmitting and receiving unit disposed on the proximal side of the probe (101).

5. The probe (101) according to Claim 4,
wherein when the plurality of transmitting and receiving units are disposed by the angles to the distal side of the probe (101), the transmitting and receiving unit disposed on the proximal side of the probe (101) is disposed by the respective angle so that a position identical to the position in the body lumen which is scanned by the signal transmitted from the other transmitting and receiving unit disposed on the distal side of the probe (101) is scanned, and
wherein when the plurality of transmitting and receiving units are disposed by the angles to the proximal side of the probe (101), the transmitting and receiving unit disposed on the distal side of the probe (101) is disposed by the respective angle so that a position identical to the position in the body lumen which is scanned by the signal transmitted from the other transmitting and receiving unit disposed on the proximal side of the probe (101) is scanned.

6. The probe (101) according to anyone of Claims 1 to 4,
wherein the plurality of transmitting and receiving units include a plurality of optical transmitting and receiving units (320) that transmit and receive light.

7. The probe (101) according to anyone of Claims 1 to 4,
wherein the plurality of transmitting and receiving units include a plurality of ultrasound transmitting and receiving units (310) that transmit and receive ultrasound.

8. An imaging apparatus for diagnosis (100) comprising:
the probe (101) according to anyone of Claims 1 to 7, and the rotary junction unit (102) to which the probe (101) is attached,
wherein the imaging apparatus for diagnosis (100) is configured to use signals transmitted and received in the probe (101) and to generate a tomographic image inside the body lumen.

## Patentansprüche

1. Sonde (101), die so konfiguriert ist, dass sie abnehmbar an einer drehbaren Verbindungseinheit (102) einer Bildgebungsvorrichtung (100) für die Diagnose angebracht ist und in eine Innenseite eines Körperlumens eingeführt werden kann, umfassend:
eine Mehrzahl von Sende- und Empfangseinheiten, die so konfiguriert sind, dass sie tomographische Bilder durch Senden und Empfangen von Signalen von der Innenseite des Körperlumens erhalten; und
eine Antriebswelle (222), die so konfiguriert ist, dass sie eine Drehantriebskraft von der drehbaren Verbindungseinheit (102) empfängt und die Mehrzahl von Sende- und Empfangseinheiten dreht,
wobei die Mehrzahl von Sende- und Empfangseinheiten jeweils entlang der axialen Richtung der Antriebswelle (222) angeordnet sind, die in der gleichen Richtung durch Winkel (a, β) in Bezug auf eine Richtung orthogonal zu der axialen Richtung der Antriebswelle (222) geneigt sind, sodass die Signale in Richtungen entweder zu einer distalen Seite der Sonde (101) oder zu einer proximalen Seite der Sonde (101) gesendet werden,
wobei jede von der Mehrzahl der Sende- und Empfangseinheiten aus einer Ultraschall-Sende- und Empfangseinheit (310), die einen Ultraschall sendet und empfängt, und einer optischen Sende- und Empfangseinheit (320), die Licht sendet und empfängt, ausgewählt ist,
wobei eine von der Mehrzahl der Sende- und Empfangseinheiten (310, 320) an der distalen Seite der Sonde (101) angeordnet ist und die andere von der Mehrzahl der Sende- und Empfangseinheiten (310, 320) an der proximalen Seite der Sonde (101) angeordnet ist, sodass ein Abstand zwischen ihnen (310, 320) L beträgt.

2. Sonde (101) nach Anspruch 1,
wobei, wenn die Mehrzahl der Sende- und Empfangseinheiten durch die Winkel so angeordnet ist, dass die Signale zu der distalen Seite der Sonde (101) gesendet werden, der Winkel der Sende- und Empfangseinheit, die auf der proximalen Seite der Sonde (101) angeordnet ist, gleich oder größer ist als der Winkel von der anderen Sende- und Empfangseinheit, die auf der distalen Seite der Sonde (101) vorgesehen ist, und
wobei, wenn die Mehrzahl der Sende- und Empfangseinheiten durch die Winkel so angeordnet ist, dass die Signale zu der proximalen Seite der Sonde (101) gesendet werden, der Winkel der Sende- und Empfangseinheit, die auf der distalen Seite der Sonde (101) angeordnet ist, gleich oder größer ist als der Winkel von der anderen Sende- und Empfangseinheit, die auf der proximalen Seite der Sonde (101) vorgesehen ist.

3. Sonde (101) nach Anspruch 1 oder 2,
wobei die Winkel von der Mehrzahl der Sende- und Empfangseinheiten identisch sind.

4. Sonde (101) nach Anspruch 2,
wobei, wenn die Mehrzahl der Sende- und Empfangseinheiten durch die Winkel zu der distalen Seite der Sonde (101) angeordnet ist, der Winkel von der Sende- und Empfangseinheit, die auf der proximalen Seite der Sonde (101) angeordnet ist, größer ist als der Winkel von der anderen Sende- und Empfangseinheit, die auf der distalen Seite der Sonde (101) angeordnet ist, und
wobei, wenn die Mehrzahl der Sende- und Empfangseinheiten durch die Winkel zu der proximalen Seite der Sonde (101) angeordnet ist, der Winkel von der Sende- und Empfangseinheit, die auf der distalen Seite der Sonde (101) angeordnet ist, größer ist als der Winkel von der anderen Sende- und Empfangseinheit, die auf der proximalen Seite der Sonde (101) angeordnet ist.

5. Sonde (101) nach Anspruch 4,
wobei, wenn die Mehrzahl der Sende- und Empfangseinheiten durch die Winkel zu der distalen Seite der Sonde (101) angeordnet sind, die Sende- und Empfangseinheit, die auf der proximalen Seite der Sonde (101) angeordnet ist, durch den entsprechenden Winkel angeordnet ist, sodass eine Position abgetastet wird, die identisch mit der Position in dem Körperlumen ist, die durch das Signal abgetastet wird, das von der anderen Sende- und Empfangseinheit, die auf der distalen Seite der Sonde (101) angeordnet ist, gesendet wird, und
wobei, wenn die Mehrzahl der Sende- und Empfangseinheiten durch die Winkel zu der proximalen Seite der Sonde (101) angeordnet sind, die Sende- und Empfangseinheit, die auf der distalen Seite der Sonde (101) angeordnet ist, durch den entsprechenden Winkel angeordnet ist, sodass eine Position abgetastet wird, die identisch mit der Position in dem Körperlumen ist, die durch das Signal abgetastet wird, das von der anderen Sende- und Empfangseinheit, die auf der proximalen Seite der Sonde (101) angeordnet ist, gesendet wird.

6. Sonde (101) nach einem der Ansprüche 1 bis 4,
wobei die Mehrzahl der Sende- und Empfangseinheiten eine Mehrzahl von optischen Sende- und Empfangseinheiten (320) umfasst, die Licht senden und empfangen.

7. Sonde (101) nach einem der Ansprüche 1 bis 4,
wobei die Mehrzahl der Sende- und Empfangseinheiten eine Mehrzahl von Ultraschall-Sende- und Empfangseinheiten (310) umfasst, die Ultraschall senden und empfangen.

8. Bildgebungsvorrichtung (100) für die Diagnose, umfassend:
die Sonde (101) nach einem der Ansprüche 1 bis 7, und die drehbare Verbindungseinheit (102), an welcher die Sonde (101) angebracht ist,
wobei die Bildgebungsvorrichtung (100) für die Diagnose so konfiguriert ist, dass sie die in der Sonde (101) gesendeten und empfangenen Signale verwendet und ein tomographisches Bild in der Innenseite des Körperlumens erzeugt.

## Revendications

1. Sonde (101) configurée pour être fixée de manière détachable à une unité de jonction rotative (102) d'un appareil d'imagerie pour diagnostic (100) et pouvant être insérée dans un intérieur d'une lumière corporelle, comprenant :
une pluralité d'unités de transmission et de réception qui sont configurées pour obtenir des images tomographiques par transmission et réception de signaux à l'intérieur de la lumière corporelle ; et
un arbre d'entraînement (222) configuré pour recevoir une force d'entraînement rotative de l'unité de jonction rotative (102) et pour amener en rotation la pluralité d'unités de transmission et de réception,
dans laquelle la pluralité d'unités de transmission et de réception sont respectivement disposées le long de la direction axiale de l'arbre d'entraînement (222) inclinée dans la même direction par des angles (α, β) par rapport à une direction perpendiculaire à la direction axiale de l'arbre d'entraînement (222) de sorte que les signaux sont transmis dans des directions soit vers un côté distal de la sonde (101) soit un côté proximal de la sonde (101),
dans laquelle chacune de la pluralité d'unités de transmission et de réception est sélectionnée à partir d'une unité de transmission et de réception d'ultrason (310) qui transmet et reçoit un ultrason et d'une unité de transmission et de réception optique (320) qui transmet et reçoit de la lumière,
dans laquelle une de la pluralité d'unités de transmission et de réception (310, 320) est disposée sur le côté distal de la sonde (101) et l'autre de la pluralité d'unités de transmission et de réception (310, 320) est disposée sur le côté proximal de la sonde (101) de sorte qu'une distance entre elles (310, 320) est L.

2. Sonde (101) selon la revendication 1,
dans laquelle lorsque la pluralité d'unités de transmission et de réception sont disposées par les angles de sorte que les signaux sont transmis vers le côté distal de la sonde (101), l'angle de l'unité de transmission et de réception disposée sur le côté proximal de la sonde (101) est supérieur ou égal à l'angle de l'autre unité de transmission et de réception prévue sur le côté distal de la sonde (101), et
dans laquelle lorsque la pluralité d'unités de transmission et de réception sont disposées par les angles de sorte que les signaux sont transmis vers le côté proximal de la sonde (101), l'angle de l'unité de transmission et de réception disposée sur le côté distal de la sonde (101) est supérieur ou égal à l'angle de l'autre unité de transmission et de réception prévue sur le côté proximal de la sonde (101).

3. Sonde (101) selon la revendication 1 ou 2,
dans laquelle les angles de la pluralité d'unités de transmission et de réception sont identiques.

4. Sonde (101) selon la revendication 2,
dans laquelle lorsque la pluralité d'unités de transmission et de réception sont disposées par les angles vers le côté distal de la sonde (101), l'angle de l'unité de transmission et de réception disposée sur le côté proximal de la sonde (101) est supérieur à l'angle de l'autre unité de transmission et de réception disposée sur le côté distal de la sonde (101), et
dans laquelle lorsque la pluralité d'unités de transmission et de réception sont disposées par les angles vers le côté proximal de la sonde (101), l'angle de l'unité de transmission et de réception disposée sur le côté distal de la sonde (101) est supérieur à l'angle de l'autre unité de transmission et de réception disposée sur le côté proximal de la sonde (101).

5. Sonde (101) selon la revendication 4,
dans laquelle lorsque la pluralité d'unités de transmission et de réception sont disposées par les angles par rapport au côté distal de la sonde (101), l'unité de transmission et de réception disposée sur le côté proximal de la sonde (101) est disposée par l'angle respectif de sorte qu'une position identique à la position dans la lumière corporelle qui est balayée par le signal transmis à partir de l'autre unité de transmission et de réception disposée sur le côté distal de la sonde (101) est balayée, et
dans laquelle lorsque la pluralité d'unités de transmission et de réception sont disposées par les angles par rapport au côté proximal de la sonde (101), l'unité de transmission et de réception disposée sur le côté distal de la sonde (101) est disposée par l'angle respectif de sorte qu'une position identique à la position dans la lumière corporelle qui est balayée par le signal transmis à partir de l'autre unité de transmission et de réception disposée sur le côté proximal de la sonde (101) est balayée.

6. Sonde (101) selon l'une quelconque des revendications 1 à 4,
dans laquelle la pluralité d'unités de transmission et de réception comportent une pluralité d'unités de transmission et de réception (320) optiques qui transmettent et reçoivent de la lumière.

7. Sonde (101) selon l'une quelconque des revendications 1 à 4,
dans laquelle la pluralité d'unités de transmission et de réception comportent une pluralité d'unités de transmission et de réception (310) d'ultrason qui transmettent et reçoivent un ultrason.

8. Appareil d'imagerie pour diagnostic (100) comprenant :
la sonde (101) selon l'une quelconque des revendications 1 à 7, et
l'unité de jonction rotative (102) à laquelle la sonde (101) est fixée,
dans lequel l'appareil d'imagerie pour diagnostic (100) est configuré pour utiliser les signaux transmis et reçus dans la sonde (101) et pour générer une image tomographique à l'intérieur de la lumière corporelle.
